# EUROPEAN PATENT SPECIFICATION

(11) **EP 1 233 932 B1**
(45) Date of publication and mention of the grant of the patent: **16.04.2008**
(21) Application number: 99953490.2
(22) Date of filing: 08.11.1999
(51) Int. Cl.: C02F 1/78

(54) **DOMESTIC METHOD AND APPARATUS FOR DISPENSING PURIFIED WATER**
VERFAHREN UND VORRICHTUNG ZUR ERZEUGUNG VON SAUBEREM WASSER IM HAUSHALT
PROCEDE ET APPAREIL DOMESTIQUE DE DISTRIBUTION D'EAU PURIFIEE

(43) Date of publication of application: 28.08.2002
(73) Proprietor: Fantom Technologies Inc., Welland, Ontario L3B 5S1 (CA)
(72) Inventor: CONRAD, Wayne, Ernest, Hampton, Ontario L0B 1J0 (CA)
(74) Representative: Harrison, David Christopher
(86) International application number: PCT/CA1999/001053
(87) International publication number: WO 2000/027761

(56) References cited:
- EP-A- 0 328 035
- WO-A-99/35096
- US-A- 4 599 166
- US-A- 4 988 484
- US-A- 5 207 993
- US-A- 5 213 773
- US-A- 5 366 619
- US-A- 5 582 717
- US-A- 5 683 576

## Description

### FIELD OF THE INVENTION

This invention relates of an apparatus for treating and dispensing water with a gas containing ozone. The apparatus may be used in the production of water fit for human consumption from water contaminated by microorganisms, chemicals, heavy metals and minerals. The gas containing ozone may be present either by itself or in combination with one or more other gasses and/or a liquid. Further, the water may be present by itself or may also have one or more liquids and/or one or more other gases associated therewith.

### BACKGROUND OF THE INVENTION

The production of water suitable for human consumption from water contaminated by one or more of microorganisms, chemicals, heavy metals and minerals is a requirement throughout the world. Many different proposals have been made for the purification of contaminated water. These include filtration, treating the water with ultraviolet light and treating the water with an oxidizing agent (eg. ozone or hydrogen peroxide). A filtration based system will typically reduce the levels of chlorine, lead, and pesticides. However, such systems are not generally designed to remove microorganisms and as such produce filtered water and not purified water. One disadvantage with ultraviolet light systems is that the ultraviolet light's disinfection efficacy is greatly diminished by turbidity or colour in the water which can cause the filter to become contaminated by microorganisms which can readily live and breed therein thereby multiplying the danger from any microorganisms which may be present.

Various processes to treat water have been developed using ozonation. For example, United States Patent No. 5,683,576 to Olsen describes an apparatus for treating contaminated water by passing ozone through the water. In the system disclosed by Olsen, an ozone containing gas is passed through the water to be treated, until the instantaneous concentration of ozone in the head space above the water being treated reaches a predetermined level. Then, the flow of ozone through the water continues for a predetermined period of time.

One disadvantage of Olsen is that the system makes the assumption that once the concentration of ozone reaches the predetermined level, that it does not subsequently drop below that level, or rise above that level.

### SUMMARY OF THE INVENTION

In some situations, the water which is received from a municipal water supply may not be of potable quality. For example, the water supply may be contaminated after it leaves a municipal water treatment plant if the supply pipes in which it is carried are corroded or broken. Further, after a natural disaster, (eg. an earthquake, flood or the like), the water supply for a municipality may be compromised by contaminants.

The instant invention provides a control system for domestic (i.e. residential) water treatment apparatus (eg. that which is used in a house or a cottage) wherein the water to be treated may be from a municipal water supply which is fed to the house through supply pipes. It may also be water which is obtained from a well maintained by the individual or any other source that the individual has for their house or cottage. The control system monitors the apparatus to determine if it is functioning according to its design specifications and advises the individual if the water is suitable for ingestion.

In accordance with the instant invention, there is provided a domestic process for treating water with an oxidizing gas in an apparatus having a dispensing system for dispensing water from a dispenser, the process comprising the steps of:
(a) initiating a treatment cycle to treat water with the oxidizing gas;
(b) during the treatment cycle, contacting the water and the oxidizing gas to effect oxidation of contaminants in the water;
(c) continuing the treatment cycle until the water is treated to a predetermined level of treatment;
(d) terminating the treatment of the water if the water is not treated to the predetermined level of treatment within a specified time;
(e) preventing the dispensing system from operating if the water is not treated to the predetermined level of treatment; and,
(f) issuing a warning signal to a user that the water has not been treated to the predetermined level of treatment if the water has not been treated to the predetermined level of treatment at the end of the treatment cycle.

The step of issuing a warning signal to the user may comprise illuminating a warning light.

In one embodiment, the process further comprises the step of automatically dispensing treated water after the water has been treated to the predetermined level of treatment when a receiving vessel is positioned beneath the dispenser and the step of issuing a warning signal to the user comprises preventing the automatic dispensing of treated water.

The apparatus may to allow the same water to be retreated by a user issuing a reactuation signal.

In another embodiment, the process further comprises the step of issuing a signal to a user that the water has been treated to the predetermined level of treatment when the cycle has been successfully completed. The step of issuing a signal to the user may comprise illuminating a light and/or automatically dispensing treated water when the cycle is successfully completed and when a receiving vessel is positioned beneath the dispenser.

In another embodiment, the process includes providing an ozone generator for generating ozone and providing the ozone as the oxidizing gas, wherein the process further comprises the step of checking that the ozone generator is operating and issuing a signal to a user when the ozone generator is working. The step of checking that the ozone generator is working may comprise reading a value from the sensor corresponding to the presence of ozone in the off gas to determine whether the ozone generator is working.

In another embodiment, the process includes providing an ozone generator for generating ozone and providing the ozone as the oxidizing gas, wherein the step of treating the water comprises treating the water until the concentration of ozone in the off gas is at least equal to a desired concentration and subsequently treating the water for an additional preset amount of time with ozone.

In another embodiment, the process includes providing an ozone generator for generating ozone, providing the ozone as the oxidizing gas and providing ozone to the water to produce an off gas which is fed to an ozone sensor, wherein the step of treating the water comprises treating the water until the amount of ozone entering the off gas is approximately equal to the amount of ozone which is being produced by the ozone generator and subsequently treating the water for an additional desired amount of time with ozone.

In another embodiment, the process further comprises the step of issuing a signal to a user that the apparatus is working.

In another embodiment, the process further comprises the steps of storing the treated water in a container and periodically retreating the water in the container.

In another embodiment, the step of preventing the dispensing system from operating may comprise the steps of:
(a) disabling the dispensing system on the occurrence of one of the following events
   (i) upon the initiation of the treatment cycle; or
   (ii) if the water has not been treated to the predetermined level of treatment- at the end of the treatment cycle; and,
(b) enabling the dispensing system if the water is treated to the predetermined level of treatment.

### BRIEF DESCRIPTTON OF THE DRAWINGS

A further, detailed description of the invention, briefly described above, will follow by reference to the following drawings of a preferred embodiment of the invention in which:
Figure 1 shows a schematic representation of an apparatus to treat water on a batch basis according to the instant invention; and,
Figure 2 shows a schematic representation of an apparatus to treat water on a continuous basis according to the instant invention.

### DESCRIPTTON OF PREFERRED EMBODIMENT

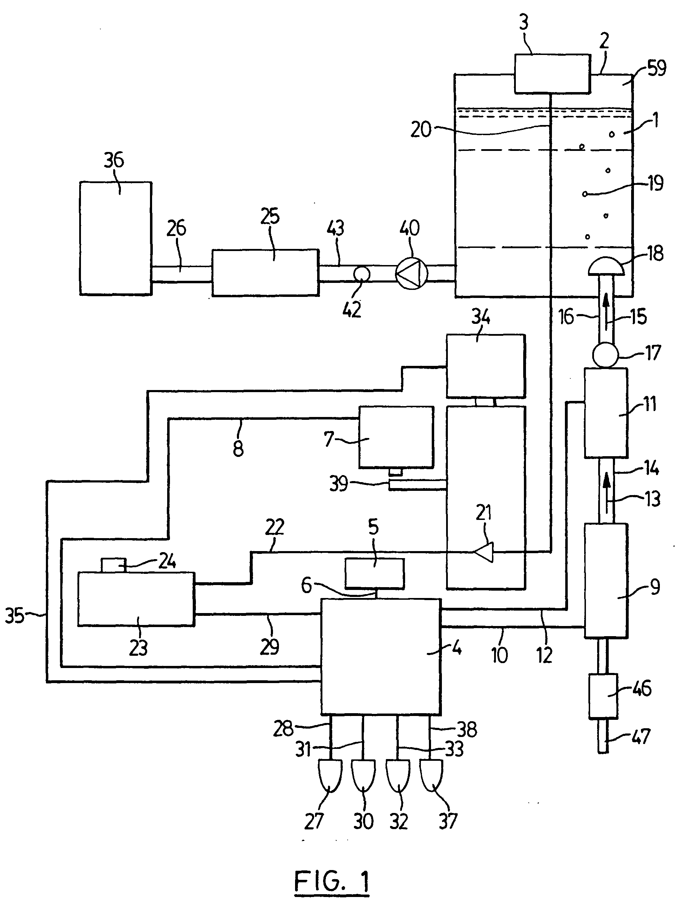

Figure 1 schematically illustrates a batch system for treating a liquid comprising water with an oxidizing gas. Preferably the liquid consists of water and the oxidizing gas is ozone either by itself or, preferably, as air containing ozone. Accordingly, the apparatus may be used for purifying and disinfecting water by means of ozone gas. The method and apparatus may be used in particular in a domestic setting for treating water prior to its use in a residence such as a counter top water treatment apparatus, a point of entry unit which is designed to treat all or some of the water which enters a dwelling or a point of use unit which is designed to treat all or some of the water exiting a tap in the dwelling. Thus, the control apparatus may be used in domestic (i.e. residential) water treatment apparatus, eg. a water treatment apparatus for use with a house, cottage, mobile home or the like. Thus, the water to be treated may be from a municipal water supply which is fed to a house through supply pipes. It may also be water which is obtained from a well maintained by the individual or any other source that the individual has for their house, cottage, mobile home or the like. It may also be used in portable water treatment equipment.

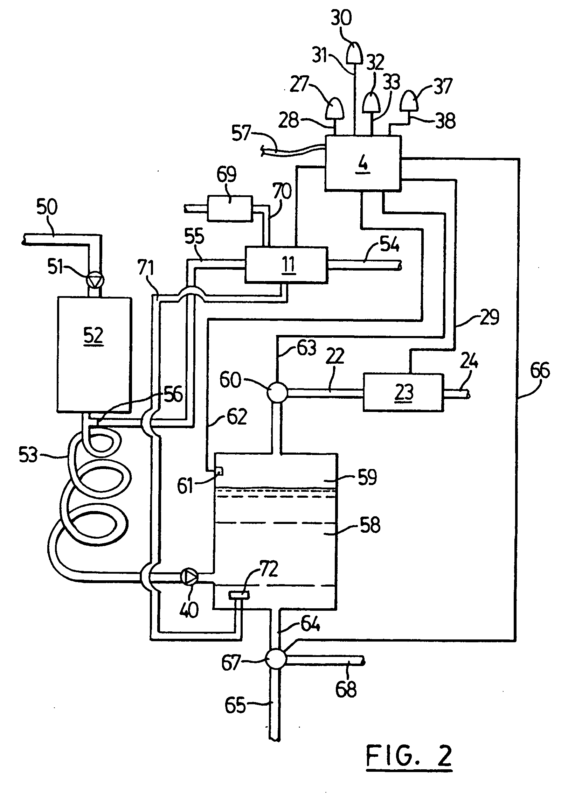

Figure 1 demonstrates a batch system. However, it is to be understood that the control system of the instant invention may be used with any domestic water treatment system (i.e. either a batch system or a continuous treatment system as exemplified in Figure 2).

In the embodiment of Figure 1, water 1 is introduced into container 2 (which may be of any particular size and shape) through an inlet port, for example a resealable cap 3. Cap 3 may be removably affixed to the container 2 by any suitable method, such as a screw thread or a bayonet mount. While container 2 may allow some of the gas to escape there from during the treatment of water 1 (eg. some of the treatment gas may exit container 2 during the treatment), the container is preferably sealed during the treatment cycle to prevent ozone from exiting the system during a treatment cycle.

The unit is provided with a source of ozone. This may be a canister of compressed ozone gas which is provided as part of the unit or fed to container 1 via a hose (not shown). Preferably, the device includes an ozone generator 11 and the ozone generator is supplied with a source of oxygen. This source of oxygen may be the ambient air (see Figure 2) or oxygen enriched air (which may be produced by an oxygen concentrator 9, such as those which utilize pressure swing adsorption and are known in the art). Pressurized air may be provided to oxygen concentrator 9 such as by a motor driven fan or air pump 46 which is supplied with ambient air by feed tube 47, or in any other manner known in the art.

In the embodiment of Figure 2, the apparatus is connected to a source of pressurized water (eg. a water pipe to a house in the case of a point of entry unit or a water pipe to a sink in the case of a point of use unit) via a feed line 50. Feed line 50 may be provided with a valve 51 to prevent a reverse flow of water out of the apparatus (eg. a check valve). The water to be treated may be passed through a filter 52 to remove suspended particulate matter as well as some dissolved minerals and metals. The water is treated in an extended contact zone such as a pipe 53 which is in fluid flow communication with gas liquid separator 58. The length of contact pipe 53 may be selected based on the degree of treatment of the water which is desired. Once the desired treatment level is determined, the length of contact pipe 53 may be determined based on the concentration of ozone introduced into the water and the flow rate of the water through contact pipe 53 so that the requisite residence time of the water in contact pipe may be obtained. Ambient air is fed to ozone generator 11 via feed tube 54. The ozone air is fed to contact pipe 53 via tube 55 and venturi 56. The venturi may be used as a source of suction to draw air into ozone generator 11. Alternately an air pump may be provided (not shown).

The controller 4 of the apparatus of Figure 1 derives power from any power source, eg. from a battery 5, by means of wire 6. The power source could also be, for example, a standard electrical outlet. For example, in the embodiment of Figure 2, controller 4 is connected to an external source of electricity via power cord 57. Power cord 57 may be plugged into a standard electrical outlet or hard wired into a household power system. A user may activate the unit by pressing the start button 7 which sends a signal to the controller 4 through the wire 8.

In the embodiment of Figure 1, when a cycle is initiated by pressing the start button, an off gas flow control valve 21 is opened, preferably by mechanical means. Preferably, the control valve 21 is opened through use of a lever 39, although any suitable device may be used. For example, a check valve may be used to open off gas tube 22. Further, upon initiation of a cycle, controller 4 preferably begins to time the cycle. Optionally, power light 27 is turned on by means of wire 28, to indicate that a cycle has been initiated.

When the unit is activated, ozone is provided to treat water 1. If the unit includes an oxygen concentrator 9 and an ozone generator 11 controller 4 provides power to oxygen concentrator 9 through wire 10. The pressurized gas containing oxygen 13 then flows from the oxygen concentrator 9 through tube 14 and into ozone generator 11 where at least a fraction of the oxygen present is converted to ozone to produce an ozone air mixture 15. Ozone generator 11 may be any ozone generator which is known in the art. This ozone air mixture 15 generated by ozone generator 11 then flows through a pipe 16, preferably through a one way check valve 17 and into container 2. The ozone air mixture may enter container 2 through a sparger 18 or any injection system or any method known in the art which serves to disperse the gas into bubbles 19. It will be appreciated that water 1 and ozone air mixture 15 may be introduced into container 2 by any method known in the art. For example, water may be introduced to container 2 via an inlet port which has a water supply (eg a hose) affixable thereto. Alternately, water 1 and mixture 2 may be introduced, either sequentially or concurrently or a combination thereof, into container 2 via a single inlet port.

After the water has been treated to the preset level, the water may be decanted from container 2. The water may be stored in container 2 until the user requires some treated water at which time it is decanted. Alternately, the water may be automatically withdrawn from container 2 at the end of the treatment cycle. The water may be withdrawn by any method known in the art. For example, the water may be withdrawn by means of pump 42. Pump 42 provides the driving force to move the treated water through tube 43, filter 25 and tube 26 into treated water carafe 36. Filter 25 may be any filter known in the water filtration art. Preferably, filter 25 comprises carbon. Carafe 36 could be removably mounted to the apparatus or it may be fixed in position and provided with an outlet (not shown) so that the water may be dispensed by means of the pressure in carafe 36, gravity or a dispensing motor. In another embodiment, container 2 could directly dispense the treated water to a user, eg. it could be removably mounted to the apparatus.

The treatment of water in the embodiment of Figure 2 is actuated when water is withdrawn from gas liquid separator 58 through outlet 64. The water in the apparatus is at an elevated pressure (which is provided by the feed source). As water is withdrawn, (eg. a tap in the house is turned on) water flows into the apparatus to replace the water which is withdrawn. As untreated water enters contact pipe 53, ozone is drawn into contact pipe 53 by means of venturi 56.

The off gas which exits the water is treated to remove ozone there from. The treated off gas may then be harmlessly vented to the ambient (eg. the room in which the apparatus is positioned). This may be achieved by filtration or a destructor which converts the ozone to a benign gas (eg. oxygen). The progress of the water treatment is monitored by a sensor. The sensor may measure the condition of the water (eg. an ORP sensor). Preferably, the sensor monitors the off gas. The embodiment of Figures 1 and 2 use an off gas sensor that preferably also acts as a destructor.

Referring to Figure 1, after passing through the water 1 in container 2, the gas exits the container 2 through a vent line 20. This removes gas from the head space in container 2 and prevents, or reduces, pressure build up in container 2. The vented gas passes preferably through an off gas flow control valve 21 (which may be a check valve), through tube 22 and to the ozone off gas sensor 23. It will be appreciated that if sensor 23 does not convert all of the ozone in the off gas to a benign gas (eg, oxygen), that an ozone destructor is preferably positioned at any position downstream of sensor 23 to convert the residual ozone to a benign gas (eg, oxygen). After ozone sensor 23, or after the ozone destructor, the gas may then be vented to the ambient via tube 24.

It will be appreciated that if container 2 is at least partially sealed, eg. valve 21 is closed or at least partially closed, that pressure will build up in container 2. The increase in pressure in container 2 is beneficial in the treatment of water 1 by mixture 15. In such a case, a bleed stream of the off gases may be passed to sensor 23. Alternately, once the pressure has built up to a desired level in container 2, a steady flow of off gasses may be removed from container 2 to prevent further pressure build up in container 2. In an alternate embodiment, valve 21 may be omitted so that all of the off gas will travel to sensor 23.

In the embodiment of Figure 2, undissolved gas exits contact pipe 53 with the treated water and is separated from the treated water by any means known in the art. A check valve 40 or other means may be provided to prevent water from flowing backwards into contact pipe 53. As shown in Figure 2, separator 58 comprises a vessel in which the gas is separated by buoyancy. The gas rises to head space 59. Pressure is maintained in separator 58 by means of valve 60. Separator 58 is provided with a pressure sensor 61 which is connected to controller 4 via wire 62. When the pressure reaches a preset limit, controller 4 signals valve 60 to open by means of wire 63. This allows some of the gas in head space 59 to travel through tube 22 to sensor 23. When the pressure drops to a preset level, pressure sensor 61 causes valve 60 to close via controller 4. Alternately, a mechanically operated valve, eg. a float switch, may be used. In the embodiment of Figure 2, air pump 69 may be provided to provide air to separator 58 via tube 70, ozone generator 11, tube 71 and sparger 72.

Signals from the ozone sensor 23 are transmitted to the controller by any means, for example a wire 29. The controller 4 receives a signal from sensor 23.

The sensor produces reading corresponding to the state of the water which is being treated. The sensor sends a signal to controller 4 to provide information to controller 4 as to the progress of the treatment cycle. This may be based on intermittent readings or continuous readings. If the sensor is a water sensor (eg. an ORP sensor) then the signal will be based on a characteristic of the water which may be correlated to the degree of treatment of the water. If the sensor is an off gas sensor, then the signal may be based on the concentration of ozone in the off gas. Ozone sensor 23 may measure the level of ozone in the off gas by any means known in the art.

Preferably, sensor 23 operates by producing a signal due to the presence of ozone in the off gas. Such sensors preferably use an ozone destructor material to convert the ozone to a benign gas, eg. oxygen. Preferably, the ozone destructor material is a catalyst is selected from one or more of manganese dioxide, titanium dioxide, iron oxide, or carbon. Most preferably, the catalyst is manganese dioxide. Of course, it will be appreciated that if the gas being detected is other than ozone, a catalyst appropriate to that gas will be selected.

For example, sensor 23 and controller 4 may operate on a CT (concentration time) basis. The ozone level may be measured on an intermittent basis as water 1 is treated but is preferably continuously monitored. Controller 4 may be programmed with a preset value corresponding to a total amount of ozone which must be measured by sensor 23 during a treatment cycle for water 1 to be treated to a desired level. Alternately, controller 4 may be programmed with a concentration/time profile (ie. the treatment profile) of ozone in the off gas which must be measured by sensor 23 for water 1 to be treated to a desired level. In the latter case, during the treatment cycle, the controller measures the amount of ozone in the off gases and compares it with preset values to monitor the progress of the water treatment.

Sensor 23 may also operate by measuring the instantaneous concentration of ozone in the off gas. For example, sensor 23 may issue a signal that the treatment of the water has been successfully completed when the amount of ozone entering the off gas is equal to the amount of ozone being produced by ozone generator 11. In such a case, if the ozone is thoroughly mixed with the water, it may be assumed that all of the material in the water which can be treated by the ozone has been rendered harmless and that the treatment cycle is complete. For example, controller 4 may be preprogrammed with the amount of ozone which will be produced by the ozone generator. When sensor 23 detects that the amount of ozone in the off gas (which may be derived from the concentration of ozone in the off gas) is the same as the preprogrammed value, then sensor 23 may issue a signal to controller 4 that the cycle has been successfully completed.

Controller 4 may also verify that ozone generator 11 is functioning properly. This may be achieved by monitoring the current drawn by ozone generator 11. If ozone generator 11 draws the anticipated current, then the controller may assume that ozone generator 11 is producing the anticipated quantity of ozone. Ozone generator monitoring could also be accomplished be measuring the output voltage from a power supply or transformer, the voltage difference (potential) between the high voltage electrode and a ground point or ground plane, or other means of electronically measuring the electrical performance of any ozone generator used. This could include measuring electrical characteristics on a control board, power supply board, or control signals issued to an ozone generator. Preferably, sensor 23 is used to determine that ozone generator 11 is operating. For example, once the operation of the treatment cycle is commenced, ozone sensor 23 may take a reading after, for example, 5 -10 seconds, which sets a base line ozone reading. The sensor then monitors the off gas for a change in the signal. If the signal changes, preferably within a preset period, then it may be assumed that ozone generator 11 is producing the anticipated quantity of ozone.

Preferably, the apparatus automatically dispenses the treated water when the treatment cycle is successfully completed. The apparatus may be in fluid flow communication with a vessel for receiving the treated water upon successful completion of the cycle. For example, in Figure 1, water pump 42 may automatically be actuated at the end of a treatment cycle to move the water into carafe 36. Alternately, the treated water carafe 36 may be a carafe that is removable from the apparatus (or may not even form part of the apparatus). The apparatus may have a switch which is opened when the treated water carafe 36 is positioned for receiving the treated water so that, upon successful completion of a treatment cycle, the treated water is automatically dispensed to the treated water carafe 36. In the embodiment of Figure 2, the water may automatically pass from outlet 64 to tube 65 which is connected to a pipe or a faucet in a house.

The apparatus preferably has a signal indicating apparatus to indicate when the water being treated should not be used, eg. if it should not be consumed. Such a signal is issued when the water has not been treated to a predetermined level of treatment when the cycle is completed (eg. the water has not been treated to kill a predetermined percent of any microorganisms in the untreated water and/or to oxidize a predetermined percent of any heavy metals or organic compounds in the untreated water). The apparatus may be designed to remove any particular amount of contaminants which may be in the untreated water. The actual level of treatment which is desired (eg. the desired state) may be inherent in the design of the equipment. However, the actual treatment level may be variable (eg. the sensor may be adjustable so that the level of purity of the treated water at which the sensor issues a signal that the treatment cycle has been successfully completed may be adjustable at the factory or in the home by the consumer). The signal that the treatment cycle has not been successfully completed and that the water should not be consumed may be that the apparatus will not dispense the treated water. For example, if the apparatus has an automatic dispensing system, then the apparatus does not automatically dispense the treated water at the end of a treatment cycle. Alternately, or in addition, if dispense button 34 must be pressed at the end of a cycle to dispense the water, then the apparatus will not dispense the treated water when the dispense button 34 is pressed. Alternately, or in addition, the signal may be a light 32 which is illuminated by means of wire 33 to indicate that the water should not be used (or a light, eg power light 27, which is illuminated during the treatment cycle and is shut off when the cycle is not successfully completed). Preferably, a positive signal is given to the user (eg. light 32 is illuminated and/or the water does not automatically dispense).

The apparatus may be prevented from dispensing the water in several ways. For example, if the apparatus has dispense button 34, then controller 4 may disable dispense button 34 upon receipt of a signal indicating that the cycle was not successfully completed (eg. controller 4 sends a signal to water pump 42 so that water pump 42 can not be actuated). If the apparatus has an automatic dispensing system, then controller 4 may not send a completion signal to enable the system to operate (eg. pump 42 may not be actuated at the end of a cycle).

More preferably, the dispensing system is disabled once start button 7 is pressed. Thus, water which is being treated may not be accidentally dispensed if the user presses dispense button 34 during the treatment cycle. Thus, controller 4 must reenable the dispensing system if the cycle is successfully completed so that the treated water may be dispensed. If the apparatus has an automatic dispensing system, then controller 4 may be set to a state when button 7 is pressed requiring a positive signal to allow the automatic dispensing system to be operated. In the embodiment of Figure 2, controller 4 may send a signal by means of wire 66 to valve 67 to cause valve 67 to divert the water to disposal tube 68 so that the water may be diverted from the household supply. For example, disposal tube may be connected in flow communication with a drain pipe (not shown).

In another embodiment, the apparatus may optionally have a signal indicating apparatus (eg. light 37 and/or the treated water may be automatically dispensed) to issue a signal to the user when the water is treated to the desired level and is ready for use. In this embodiment, sensor 23 may issue a signal that the treatment of the water has been successfully completed. For example, if the off gas concentration rises as expected (eg. in accordance with a preset range of values or in accordance with a preprogrammed concentration of ozone versus time profile in the off gas or the ozone concentration reaches a preset concentration for the preset time within a preset time period), or if sensor 23 detects a preset level of ozone in the off gas (eg. which is approximately the same as the concentration produced by ozone generator 11), controller 4 detects this as a successful completion of the water treatment process and may then signal the user that the cycle was successfully completed. To this end, controller may shut down the power to power light 27 by means of wire 28, the power to oxygen concentrator 9 by means of wire 10, and the power to ozone generator 11 by means of wire 12. It may also cause the water ready light 37 to be illuminated by means of wire 38. Alternately, or in addition, the dispensing system may be enabled. Accordingly, depressing water dispense button 34 will send a signal to the controller 4 by means of wire 35 and will cause the treated water to be decanted from container 2. If the apparatus has an automatic dispensing system, the water may be automatically dispensed at the end of the treatment cycle.

If the amount of ozone flowing into the vessel reaches the requisite level more quickly than expected (eg. the concentration of ozone in the off gas rises too rapidly in relation to preset control values), a problem may exist with the dispersion of the ozone containing gas throughout the contaminated water. That is, the ozone may be passing through the water in a localized area (eg. the sparger may be broken and releasing larger bubbles into the water to be treated). In such a circumstance, the apparatus may also optionally include a system fault light 30 which when illuminated by means of wire 31 indicates to the user that the water has not been fully treated and that the apparatus should be checked to ensure it is in proper working order. Controller 4 may detect this as a fault and may shut down the power to power light 27 (for example by means of wire 28), the power to oxygen concentrator 9 through wire 10, and the power to ozone generator 11 through wire 12. As a safety feature in this circumstance, depressing water dispense button 34 will not cause water to be dispensed from container 2 (eg. button 34 may send a signal to the controller 4 by means of wire 35 but the input will have no effect as the existence of a system fault precludes permission to dispense the water from the container 2 to the treated water carafe 36). Light 30 will advise the user to, eg., manually empty container 2 and that the unit may require servicing.

In another embodiment, if, after a predetermined length of time, no ozone has been detected in the treatment vessel, controller 4 may also be programmed to issue an indication to the user to check ozone generator 11 to ensure that it is working properly. For example, if the off gas concentration rises too slowly in relation to preset control values (eg. too slowly compared to a preset range of values or slower than a preprogrammed concentration of ozone versus time profile in the off gas or it does not reach a preset concentration within a preset time period or it is detected that ozone generator 11 is not operating), controller 4 may detect this as a fault and may similarly shut down the power to the power light 27 by means of wire 28, the power to the oxygen concentrator 9 through wire 10, and the power to the ozone generator 11 through wire 12. It may also cause bad water light 32 to be illuminated by means of wire 33. Also as a safety feature in this circumstance, depressing the water dispense button 34 may send a signal to the controller 4 by means of wire 35 but the input will have no effect as the existence of an unsatisfactory treatment condition indicated to the user by light 32 being activated precludes water from being dispensed from the treatment vessel by the dispensing system.

In the batch treatment of water, controller 4 also preferably controls the flow of ozone through the water being treated. When the water has been treated to the desired level, the flow of ozone through the water is stopped (eg. ozone generator 11 may be deactivated). The air pump 46 may continue to run for a period of time (eg. thirty seconds to one minute) to flush ozone from the head space 59. Alternately, or in addition, air pump 46 may operate when the water is dispensed from container 2 so as to assist in the dispensing operation.

In a preferred embodiment, controller 4 may also include a timer. The timer is used to require the apparatus to treat the water with ozone for an additional amount of time (eg. thirty seconds to one minute) once sensor 23 sends a signal indicating that the treatment cycle has been successfully completed (eg. that all of the ozone being produced by the ozone generator is entering the off gas). This additional amount of treatment time provides an additional assurance to the user that the water has been fully treated. In such a case, light 37 is preferably illuminated after the additional processing time set by the timer.

Controller 4 may also include a second timer. This timer monitors the duration of the entire treatment cycle (eg. from the initiation of the treatment cycle by pushing start button 7). In this embodiment, once a preset amount of time has elapsed during the treatment cycle, controller 4 terminates the treatment of water 1 (eg. power to ozone generator 11 may be terminated). The termination of the treatment cycle indicates that the water still has material in it that has not been treated (eg. not all of the ozone is passing through the water) and that the water should not be consumed. The apparatus may allow the user to depresses start button 7 thereby recommencing the treatment cycle (eg. when the user depresses start button 7, the timer may be reset). Alternately, the apparatus may not allow the treatment cycle to be recommenced until the user replaces the water in the treatment vessel (eg. the apparatus may be reset when container 2 is removed indicating the container 2 has been removed from the apparatus to permit it to be emptied and refilled).

In a further embodiment, the water may be automatically retreated if it is not dispensed from the apparatus, but remains in a storage vessel, to maintain the quality of the treated water. For example, if the water is allowed to stand for too long in the apparatus after treatment, then microorganisms may commence growing. To this end controller 4 preferably also reactuates ozone generator 11 to provide ozone to the treated water on a periodic basis after the successful completion of a treatment cycle. Thus, in the embodiment of Figure 1, air pump 46 and ozone generator 11 may be actuated for (eg. 15 seconds) until the water is removed from container 2 or for a set period of time (eg. 3 hours). Thus if water is dispensed on a glass by glass basis, the water will be ready for use at all times. After a predetermined period of time (eg.3 hours) controller may prevent any water remaining in the treatment vessel from being dispensed until a further treatment cycle is conducted to treat water to a predetermined level of treatment. This could comprises retreating the water in the treatment vessel or the user filling the treatment vessel and initiating a treatment cycle.

The following modification may be made: lights 27, 30, 32 and 37 may signal a user by issuing any audio, visual or other signal which will advise a user of the specified condition. Each light may be capable of being illuminated to appear different colours so as to send alternating signals to the user thus reducing the total number of lights which are required.

## Claims

1. A domestic process for treating water with an oxidizing gas in an apparatus having a dispensing system for dispensing water from a dispenser, the process comprising the steps of:
(a) initiating a treatment cycle to treat water with the oxidizing gas;
(b) during the treatment cycle, contacting the water and the oxidizing gas to effect oxidation of contaminants in the water;
(c) continuing the treatment cycle until the water is treated to a predetermined level of treatment;
(d) terminating the treatment of the water if the water is not treated to the predetermined level of treatment within a specified time;
(e) preventing the dispensing system from operating if the water is not treated to the predetermined level of treatment; and,
(f) issuing a warning signal to a user that the water has not been treated to the predetermined level of treatment if the water has not been treated to the predetermined level of treatment at the end of the treatment cycle.

2. The process as claimed in claim 1 further comprising the step of automatically dispensing treated water after the water has been treated to the predetermined level of treatment when a receiving vessel is positioned beneath the dispenser and the step of issuing a warning signal to the user comprises preventing the automatic dispensing of treated water.

3. The process as claimed in claim 1 further comprising the step of enabling the apparatus to allow the same water to be retreated by a user issuing a reactuation signal.

4. The process as claimed in claim 1 further comprising the step of issuing a signal to a user that the water has been treated to the predetermined level of treatment when the cycle has been successfully completed.

5. The process as claimed in claim 4 wherein the step of issuing a signal to the user comprises automatically dispensing treated water when the cycle is successfully completed and when a receiving vessel is positioned beneath the dispenser.

6. The process as claimed in claim 1 which includes providing an ozone generator for generating ozone and reading a value from an ozone sensor corresponding to the presence of ozone in the off gas to determine whether the ozone generator is working properly.

7. The process as claimed in claim 1 which includes providing an ozone generator for generating ozone and providing the ozone as the oxidizing gas, wherein the step of treating the water comprises treating the water until the concentration of ozone in the off gas is at least equal to a desired concentration and subsequently treating the water for an additional preset amount of time with ozone.

8. The process as claimed in claim 1 which includes providing an ozone generator for generating ozone, providing the ozone as the oxidizing gas and providing ozone to the water to produce an off gas which is fed to an ozone sensor, wherein the step of treating the water comprises treating the water until the amount of ozone entering the off gas is approximately equal to the amount of ozone which is being produced by the ozone generator and subsequently treating the water for an additional desired amount of time with ozone.

9. The process as claimed in claim 1 further comprising the steps of storing the treated water in a container and periodically retreating the water in the container.

10. The process as claimed in claim 1 wherein the step of preventing the dispensing system from operating comprises the steps of:
(a) disabling the dispensing system on the occurrence of one of the following events
(i) upon the initiation of the treatment cycle; or
(ii) if the water has not been treated to the predetermined level of treatment at the end of the treatment cycle; and,
(b) enabling the dispensing system if the water is treated to the predetermined level of treatment.

11. The process as claimed in any of claims 1-10 wherein the oxidizing gas comprises ozone and the process further comprises the steps of:
(a) treating the water with ozone and obtaining treated water and an off gas stream comprising ozone;
(b) treating at least a portion of the ozone in off gas stream to produce a signal indicative of the level of treatment of the water; and,
(c) using the signal to control the treatment of the water.

12. The process as claimed in any of claims 1-11 wherein the oxidizing gas comprises ozone and the process further comprises further the step of treating the water for an additional preset amount of time with ozone after the water has been treated to the predetermined level of treatment.

## Patentansprüche

1. Verfahren zur Behandlung von Wasser im Haushalt mit einem Oxidationsgas in einer Vorrichtung mit einem Abgabesystem zur Abgabe von Wasser aus einer Abgabevorrichtung, wobei das Verfahren folgende Schritte umfasst:
(a) Beginnen eines Behandlungszyklus zur Behandlung von Wasser mit dem Oxidationsgas;
(b) Kontaktieren des Wassers mit dem Oxidationsgas während des Behandlungszyklus, um Kontaminanten im Wasser zu oxidieren;
(c) Fortsetzen des Behandlungszyklus bis das Wasser bis zu einem vorbestimmten Behandlungsgrad behandelt wird;
(d) Beenden der Behandlung des Wassers, wenn das Wasser innerhalb einer bestimmten Zeit nicht bis zum vorbestimmten Behandlungsgrad behandelt wird;
(e) Verhindern des Betriebs des Abgabesystems, wenn das Wasser nicht bis zum vorbestimmten Behandlungsgrad behandelt wird; und
(f) Ausgeben eines Warnsignals an einen Benutzer, dass das Wasser nicht bis zum vorbestimmten Behandlungsgrad behandelt worden ist, wenn das Wasser am Ende des Behandlungszyklus nicht bis zum vorbestimmten Behandlungsgrad behandelt worden ist.

2. Verfahren nach Anspruch 1, ferner umfassend den Schritt der automatischen Abgabe von behandeltem Wasser nachdem das Wasser bis zum vorbestimmten Behandlungsgrad behandelt worden ist, wenn ein Aufnahmegefäß unterhalb der Abgabevorrichtung positioniert wird und den Schritt des Ausgebens eines Warnsignals an den Benutzer umfasst, dass die automatische Abgabe von behandeltem Wasser verhindert wird.

3. Verfahren nach Anspruch 1, ferner umfassend den Schritt zum Freigeben der Vorrichtung, um zu ermöglichen, dass das gleiche Wasser durch einen Benutzer, der ein Wiederbetätigungssignals abgibt, erneut behandelt wird.

4. Verfahren nach Anspruch 1, ferner umfassend den Schritt des Ausgebens eines Signals an einen Benutzer, dass das Wasser bis zum vorbestimmten Behandlungsgrad behandelt worden ist, wenn der Zyklus erfolgreich beendet wurde.

5. Verfahren nach Anspruch 4, worin der Schritt des Ausgebens eines Signals an den Benutzer die automatische Abgabe von behandeltem Wasser umfasst, wenn der Zyklus erfolgreich beendet wird und wenn ein Aufnahmegefäß unterhalb der Abgabevorrichtung positioniert wird.

6. Verfahren nach Anspruch 1, umfassend die Bereitstellung eines Ozongenerators zur Erzeugung von Ozon und das Ablesen eines Ozonsensorwerts, der der Gegenwart von Ozon in dem Abgas entspricht, um zu bestimmen, ob der Ozongenerator richtig arbeitet.

7. Verfahren nach Anspruch 1, umfassend die Bereitstellung eines Ozongenerators zum Erzeugen von Ozon und die Bereitstellung des Ozons als Oxidationsgas, worin der Schritt des Behandelns des Wassers die Behandlung von Wasser umfasst, bis die Ozonkonzentration in dem Abgas zumindest einer gewünschten Konzentration entspricht, sowie die anschließende Behandlung des Wassers mit Ozon über eine zusätzliche festgelegte Zeitdauer umfasst.

8. Verfahren nach Anspruch 1, umfassend die Bereitstellung eines Ozongenerators zum Erzeugen von Ozon, die Bereitstellung des Ozons als Oxidationsgas sowie die Bereitstellung von Ozon für das Wasser, um ein Abgas zu bilden, das einem Ozonsensor zugeführt wird, worin der Schritt des Behandelns des Wassers die Behandlung von Wasser umfasst, bis die in das Abgas eintretende Ozonmenge ungefähr gleich der Ozonmenge ist, die vom Ozongenerator erzeugt wird, sowie die anschließende Behandlung des Wassers mit Ozon über eine zusätzliche gewünschte Zeitdauer umfasst.

9. Verfahren nach Anspruch 1, ferner umfassend die Schritte des Lagerns des behandelten Wassers in einem Behälter und der regelmäßigen Wiederbehandlung des Wassers in dem Behälter.

10. Verfahren nach Anspruch 1, worin der Schritt, der den Abgabesystembetrieb verhindert, folgende Schritte umfasst:
(a) Inaktivieren des Abgabesystems, wenn eines der nachstehenden Ereignisse eintritt
(i) Beginn des Behandlungszyklus; oder
(ii) wenn das Wasser am Ende des Behandlungszyklus nicht bis zum vorbestimmten Behandlungsgrad behandelt worden ist; und
(b) Freigeben des Abgabesystems, wenn das Wasser bis zum vorbestimmten Behandlungsgrad behandelt wird.

11. Verfahren nach einem der Ansprüche 1 bis 10, worin das Oxidationsgas Ozon umfasst und das Verfahren ferner die folgenden Schritte umfasst:
(a) Behandeln des Wassers mit Ozon und Erhalten von behandeltem Wasser und eines Abgasstroms der Ozon umfasst;
(b) Behandeln zumindest eines Anteils des Ozons im Abgasstrom, um ein Signal zu erzeugen, das den Behandlungsgrad des Wassers anzeigt; und
(c) Verwenden des Signals zur Steuerung der Wasserbehandlung.

12. Verfahren nach einem der Ansprüche 1 bis 11, worin das Oxidationsgas Ozon umfasst und das Verfahren ferner den Schritt des Behandelns des Wassers mit Ozon über eine zusätzliche festgelegte Zeitdauer umfasst, nachdem das Wasser bis zum vorbestimmten Behandlungsgrad behandelt worden ist.

## Revendications

1. Procédé domestique pour traiter l'eau avec un gaz oxydant dans un appareil possédant un système de distribution pour distribuer l'eau d'un distributeur, le procédé comprenant les étapes consistant à:
(a) initier un cycle de traitement pour traiter l'eau avec le gaz oxydant;
(b) pendant le cycle de traitement, mettre en contact l'eau et le gaz oxydant pour effectuer l'oxydation des contaminants dans l'eau;
(c) continuer le cycle de traitement jusqu'à ce que l'eau soit traitée à un niveau de traitement prédéterminé;
(d) terminer le traitement de l'eau si l'eau n'est pas traitée au niveau de traitement prédéterminé dans un temps spécifié;
(e) empêcher le fonctionnement du système de distribution si l'eau n'est pas traitée au niveau de traitement prédéterminé; et
(f) émettre un signal d'avertissement à un utilisateur que l'eau n'a pas été traitée au niveau de traitement prédéterminé si l'eau n'a pas été traitée au niveau de traitement prédéterminé à la fin du cycle de traitement.

2. Procédé selon la revendication 1, comprenant en outre l'étape consistant à distribuer automatiquement l'eau traitée après que l'eau a été traitée à un niveau de traitement prédéterminé lorsqu'un récipient est positionné en dessous du distributeur et l'étape consistant à émettre un signal d'avertissement à l'utilisateur comprend l'empêchement de la distribution automatique de l'eau traitée.

3. Procédé selon la revendication 1, comprenant en outre l'étape consistant à permettre à l'appareil de permettre le retraitement de la même eau par un utilisateur émettrant un signal de réactionnement.

4. Procédé selon la revendication 1, comprenant en outre l'étape consistant à émettre un signal à un utilisateur que l'eau a été traitée au niveau de traitement prédéterminé lorsque le cycle a été achevé avec succès.

5. Procédé selon la revendication 4, où l'étape d'émission d'un signal à l'utilisateur comprend la distribution automatique de l'eau traitée lorsque le cycle a été achevé avec succès et lorsqu'un récipient est placé en dessous du distributeur.

6. Procédé selon la revendication 1, qui comprend la réalisation d'un générateur d'ozone pour engendrer de l'ozone et pour lire une valeur d'un capteur d'ozone correspondant à la présence d'ozone dans les gaz de dégagement pour déterminer si le générateur d'ozone fonctionne correctement.

7. Procédé selon la revendication 1, qui comprend la réalisation d'un générateur d'ozone pour produire de l'ozone et pour fournir l'ozone comme gaz oxydant, où l'étape consistant à traiter l'eau comprend le traitement de l'eau jusqu'à ce que la concentration d'ozone dans les gaz de dégagement soit au moins égale à une concentration souhaitée et à traiter ensuite l'eau pendant une période de temps additionnelle préréglée avec de l'ozone.

8. Procédé selon la revendication 1, qui comprend la réalisation d'un générateur d'ozone pour produire de l'ozone, la réalisation de l'ozone comme gaz oxydant et la fourniture de l'ozone à l'eau pour produire un gaz de dégagement qui est amené à un capteur d'ozone, où l'étape de traitement de l'eau comprend le traitement de l'eau jusqu'à ce que la quantité d'ozone entrant dans le gaz de dégagement soit approximativement égale à la quantité d'ozone qui est produite par le générateur d'ozone et à traiter ensuite l'eau pendant une durée de temps additionnelle souhaitée avec de l'ozone.

9. Procédé selon la revendication 1, comprenant en outre les étapes consistant à stocker l'eau traitée dans un contenant et à retraiter périodiquement l'eau dans le contenant.

10. Procédé selon la revendication 1, où l'étape consistant à empêcher le fonctionnement du système de distribution comprend les étapes consistant à:
(a) invalider le système de distribution lors de la survenue d'un des évènements suivants
(i) lors de l'initiation du cycle de traitement; ou
(ii) si l'eau n'a pas été traitée au niveau de traitement prédéterminé à la fin du cycle de traitement; et,
(b) valider le système de distribution si l'eau est traitée au niveau de traitement prédéterminé.

11. Procédé selon l'une des revendications 1 à 10, où le gaz oxydant comprend de l'ozone, et le procédé comprend en outre les étapes consistant à:
(a) traiter l'eau avec l'ozone et obtenir de l'eau traitée et un écoulement de gaz de dégagement comprenant l'ozone;
(b) traiter au moins une portion de l'ozone dans l'écoulement de gaz de dégagement pour produire un signal indicatif du niveau de traitement de l'eau; et
(c) utiliser le signal pour contrôler le traitement de l'eau.

12. Procédé selon l'une des revendications 1 à 11, où le gaz oxydant comprend l'ozone, et le procédé comprend en outre l'étape consistant à traiter l'eau pendant une durée de temps additionnelle préréglée avec l'ozone après que l'eau a été traitée au niveau de traitement prédéterminé.
